# EUROPEAN PATENT SPECIFICATION

(11) **EP 3 215 106 B1**
(45) Date of publication and mention of the grant of the patent: **03.04.2019**
(21) Application number: 15823084.7
(22) Date of filing: 17.12.2015
(51) Int. Cl.: A61K 8/27, A61Q 11/00, A61K 8/97

(54) **ORAL CARE COMPOSITION AND METHOD OF USE**
MUNDPFLEGEMITTEL UND VERWENDUNG
COMPOSITION DE SOIN BUCCAL ET PROCÉDÉ D'UTILISATION

(30) Priority: 23.12.2014 IN 3884DE2014
(43) Date of publication of application: 13.09.2017
(73) Proprietor: Colgate-Palmolive Company, New York, NY 10022 (US)
(72) Inventor: SIRDESAI, Amit U, Mumbai 4000080 (IN); IYER, Divya, Mumbai 400071 (IN); PLATA, Rolando, Las Pinas City PH (US); POTNIS, Shashank, Thane (W) 400610 (IN)
(74) Representative: Wibbelmann, Jobst
(86) International application number: PCT/US2015/066326
(87) International publication number: WO 2016/106072

(56) References cited:
- EP-A1- 2 689 805
- WO-A1-2005/123101
- WO-A1-2007/042472

## Description

### BACKGROUND

Mouth ulcers or canker sores are caused by various reasons including but not limited to medication side effects, chemotherapy, mechanical injury, brushing, toothpaste, food allergy, smoking, tobacco, alcohol, acidity, lack of sleep, bacterial/viral/fungal infections, and dental braces. There are various products currently sold for treatment of mouth ulcers, which include antibiotics such as doxycyline, local anesthetics such as lidocaine in combination with an antibacterial agent such as cetrimide, and an astringent such as tannic acid in combination with an anti-inflammatory agent (such as choline salicylate. Access to these formulations typically requires a prescription from physician, and frequent usage of antibiotics may lead antibiotic resistance.

Use of products that do not contain antibiotics is desirable to many consumers. Such products also are desirable for sustainability reasons. Thus, there is a strong need for such products for treating mouth ulcers.

### BRIEF SUMMARY

The present disclosure provides oral care compositions for treatment of mouth ulcers and canker sores. The compositions comprise Amla extract, Liquorice Extract, a source of zinc, and camphor. In some embodiments, the compositions further include an essential oil extract such as eugenol. In further embodiments, the compositions include one or more of thickeners, solvents, preservatives, sweeteners, colorants and/or additional excipients. Methods of treating ulcerous conditions of the mouth using the compositions are also provided.

Further areas of applicability of the present invention will become apparent from the detailed description provided hereinafter. It should be understood that the detailed description and specific examples, while indicating the preferred embodiment of the invention, are intended for purposes of illustration only and are not intended to limit the scope of the invention.

### DETAILED DESCRIPTION

The following description of the preferred embodiment(s) is merely exemplary in nature and is in no way intended to limit the invention, its application, or uses.

As used throughout, ranges are used as shorthand for describing each and every value that is within the range. Any value within the range can be selected as the terminus of the range. In addition, all references cited herein are hereby incorporated by referenced in their entireties. In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts given are based on the active weight of the material.

The inventors have found that the components of Amla extract and Liquorice (or Licorice) extract, when combined with a source of zinc ions, can be formulated into an oral care composition that possesses antimicrobial properties comparable or superior to existing oral care compositions. Thus, the present disclosure provides oral care compositions for treatment of ulcerous conditions of the mouth, such as mouth ulcers and canker sores. In a first embodiment, the present disclosure provides an oral care composition (Composition 1) comprising Amla extract, Liquorice Extract, a zinc ion source, and optionally camphor, in a pharmaceutically acceptable carrier; for example:
1.1. Composition 1, wherein the composition is an oral gel.
1.2. Composition 1 or 1.1, wherein the Amla extract is present in the composition in an amount of from 0.5% to 8% by weight, for example from 1% to 5% by weight, for example 1% or 5% by weight; the Liquorice extract is present in the composition in an amount of from 0.1% to 5% by weight, for example from 1% to 3% by weight, for example 1% or 2.5% by weight; the zinc source is present in the composition in an amount sufficient to provide zinc ions in an amount of from 0.1% to 2% by weight, for example from 0.5% to 1.2% by weight, for example about 0.8% by weight.
1.3. Any preceding Composition 1 et seq., wherein the Amla extract is present in the composition in an amount of from 0.5% to 8% by weight; the Liquorice extract is present in the composition in an amount of from 0.1% to 5% by weight; the zinc source is present in the composition in an amount sufficient to provide zinc ions in an amount of from 0.1% to 2% by weight; and the camphor, is present in the composition in an amount of from 0% to 2% by weight.
1.4. Any preceding Composition 1 et seq., wherein the Amla extract is present in the composition in an amount of from 1% to 5% by weight; the Liquorice extract is present in the composition in an amount of from 1% to 3% by weight; and the zinc source is present in the composition in an amount sufficient to provide zinc ions in an amount of from 0.5% to 1.2% by weight.
1.5. Any preceding Composition 1 et seq., wherein the zinc ion source is zinc acetate, zinc citrate, zinc gluconate, zinc glycinate, zinc oxide, zinc sulfate and sodium zinc citrate, or a combination of two or more thereof; preferably zinc oxide or zinc sulfate or a combination thereof.
1.6. Any preceding Composition 1 et seq., wherein the zinc ion source is zinc oxide or zinc sulfate, or a combination thereof.
1.7. Composition 1.5 or 1.6, wherein the zinc oxide is in particulate form.
1.8. Any preceding oral care Composition 1 et seq., further comprising one or more flavorants, for example one or more of eugenol, L-menthol, clove oil, thymol, coriander oil, eucalyptus oil, fennel oil, neem oil, lemon oil, coconut oil and/or basil oil.
1.9. Any preceding oral care Composition 1 et seq., wherein eugenol is present in an amount of from 0.1% to 2%, for example 0.1% to 1%, for example 0.6%; and L-menthol is present in an amount of from 0.1% to 4%, for example 0.1% to 2%, for example 1% by weight of the composition.
1.10. Any preceding oral care Composition 1 et seq., wherein eugenol is present in an amount of from 0.1% to 2%; and L-menthol is present in an amount of from 0.1% to 4%, by weight of the composition.
1.11. Any preceding oral care Composition 1 et seq., wherein eugenol is present in an amount of from 0.1% to 1%; and L-menthol is present in an amount of from 0.1% to 2%, by weight of the composition.
1.12. Any preceding oral care Composition 1 et seq., wherein clove oil is present in an amount of from 0.1% to 2%, for example 0.1% to 1%, for example 0.6%; and L-menthol is present in an amount of from 0.1% to 4%, for example 0.1% to 2%, for example 1% by weight of the composition.
1.13. Any preceding oral care Composition 1 et seq., wherein clove oil is present in an amount of from 0.1% to 2%; and L-menthol is present in an amount of from 0.1% to 4%, by weight of the composition.
1.14. Any preceding oral care Composition 1 et seq., wherein clove oil is present in an amount of from 0.1% to 1%; and L-menthol is present in an amount of from 0.1% to 2%, by weight of the composition.
1.15. Any preceding oral care Composition 1 et seq., wherein coconut oil is present in an amount of from 0.1% to 5%, for example 1% to 4%, for example 3% by weight of the composition.
1.16. Any preceding oral care Composition 1 et seq., wherein coconut oil is present in an amount of from 1% to 4%, by weight of the composition.
1.17. Any preceding oral care Composition 1 et seq., further comprising clove oil, in an amount of from 0.1% to 4% by weight of the composition.
1.18. Any preceding Composition 1 et seq., further comprising one or more thickeners.
1.19. Composition 1.18, wherein the thickeners are selected from modified celluloses, for example hydroxyethylcellulose and carboxymethylcellulose, PMC, Veegum, Xanthan, hydropropylcellulose, methylcellulose, carrageenan, tragacanth and combinations of two or more thereof.
1.20. Any preceding Composition 1 et seq., further comprising a nonionic surfactant.
1.21. Composition 1.20, wherein the nonionic surfactant is selected from Croduret 40, a PEG, PEG 600, polysorbate 20 (Tween™ 20), polysorbate 80 (Tween™ 80), PEG 6000, Poloxamers, and combinations of two or more thereof.
1.22. Composition 1.20, wherein the nonionic surfactant is polysorbate 20 (Tween™ 20), alone or in combination with another nonionic surfactant.
1.23. Any preceding Composition 1 et seq., further comprising one or more solvents.
1.24. Composition 1.12, wherein the solvent or solvents are selected from glycerin, propylene glycol, Croduret 40, PEGs, PEG 600, Tween 80, PEG 6000, and Poloxamers.
1.25. Composition 1.23, wherein the solvents comprise propylene glycol.
1.26. Any preceding Composition 1 et seq., further comprising one or more humectants, for example glycerin.
1.27. Any preceding Composition 1 et seq., further comprising one or more preservatives.
1.28. Composition 1.27, wherein the preservatives are selected from sodium benzoate, methyl paraben, propyl paraben, benzyl alcohol, ethanol, citric acid, potassium sorbate, salicylic acid, sorbic acid, inorganic sulfites, triclosan, flavors and combinations of two or more thereof.
1.29. Any preceding Composition 1 et seq., further comprising one or more additional ingredients selected from sweeteners and/or colorants, for example stevia, sucralose, liquorice, aspartame sodium, zinc oxide, talc, titanium dioxide, pigments, dyes and combinations of two more more thereof.

The present disclosure further provides a method (Method 1) for treating ulcerous conditions of the oral cavity, for example mouth ulcers and canker sores, comprising administering to a patient in need thereof a therapeutically effective amount of a composition of any of Compositions 1-1.29.

The present disclosure further provides a method (Method 2) for preparing a composition as described above (e.g., Composition 1-1.29) comprising combining together Amla extract, Liquorice Extract, a zinc ion source, and camphor, in a pharmaceutically acceptable carrier.

The present disclosure further provides the use of the combination of Amla extract, Liquorice Extract, a zinc ion source, and camphor, in the preparation of a medicament for treating ulcerous conditions of the oral cavity, for example mouth ulcers and canker sores.

The compositions of the invention contain Amla extract (*Embilica Officinalis),* also known as Indian Gooseberry. Amla extract contains 40% tannins, which provide astringency. Indian gooseberry has undergone preliminary research, demonstrating in vitro antiviral and antimicrobial properties. There is preliminary evidence *in vitro* that its extracts induce apoptosis and modify gene expression in osteoclasts involved in rheumatoid arthritis and osteoporosis. It may prove to have potential activity against some cancers. Experimental preparations of leaves, bark or fruit have shown potential efficacy against laboratory models of disease, such as for inflammation, cancer, age-related renal disease, and diabetes.

Amla extract is also known for its antioxidant properties, and is reported to have stronger antioxidant properties than ascorbic acid. See Khopde et al., Current Science 81 (2) 2001, incorporated herein by reference for all purposes. While Amla is perceived to be rich in vitamins, and particularly vitamin C, the content of vitamins is in microgram level, and the antioxidant effect is mainly due to the higher percentage of tannins. While not wishing to limit the invention, it is theorized that vitamin C is not oxidized due to the high percentage of tannins, which act as antioxidant, thus making the vitamin available for consumption. Methods of preparing Amla extract can be found in, for example, Khopde et al., *supra.*

The Amla extracts suitable for inclusion in the compositions of the invention can be prepared by any of a wide variety of procedures. Amla extracts suitable for inclusion in the compositions of the invention preferably do not contain any solvents or preservatives. In one commercial procedure, fresh Emblica officinalis fruits are pulped at less than 5°C to produce Amla pulp, which is then subjected to cold press extraction to produce Amla juice, and filtered to remove any insoluble matter. The juice is concentrated under vacuum, and spray dried and formulated, for example with colloidal silicon dioxide. The product is then milled and sifted. Suitable Amla extracts prepared by such a procedure can be purchased from, or example, Sabinsa Corporation, East Windsor, NJ.

The oral care compositions of the present disclosure also contain Liquorice extract. Liquorice extracts have been extensively used for their therapeutic properties. The activities of Liquorice extracts, including wound healing properties, have been linked to different classes of phytochemicals, particularly the major water-soluble constituent glycyrrhizin and its hydrolysis product 18β-glycyrrhetinic acid. Liquorice extract has been used in various products for ulcer treatment. It is postulated that glycyrrhetinic Acid has a structure similar to cortisone and hence provides ant-inflammatory properties.

The liquorice extracts suitable for inclusion in the compositions of the invention can be prepared by any of a wide variety of procedures. Liquorice extracts suitable for inclusion in the compositions of the invention preferably do not contain any solvents or preservatives. In one commercial procedure, liquorice root powder is extracted with water, concentrated, and acidified and filtered. The residue is washed with water until neutral pH is achieved, and then dried, milled and sifted. Suitable Liquorice extracts prepared by such a procedure can be purchased from, or example, Sabinsa Corporation, East Windsor, NJ.

The oral care compositions of the present disclosure also contain a source of zinc ions, useful, for example, as an antimicrobial, anticalculus or breath-freshening agents. One or more such sources can be present. Suitable zinc ion sources include without limitation zinc acetate, zinc citrate, zinc gluconate, zinc glycinate, zinc oxide, zinc sulfate, sodium zinc citrate and the like. One or more zinc ion sources are optionally and illustratively present in a total amount of 0.05% to 3%, for example 0.1% to 1%, by weight of the composition. Preferably, the amount of zinc salt is chosen to provide zinc ions in an amount of from 0.1% to 2% by weight. For example from 0.5% to 1.2% by weight, for example about 0.8% by weight. For example, zinc sulphate used at 2.2% by weight provides elemental Zinc (ions) of about 0.8% by weight, and Zinc Oxide used at 1% by weight provides elemental Zinc (ions) of about 0.8% by weight.

In some preferred embodiments, the compositions include Zinc Oxide and/or Zinc Sulfate, each of which is known for its astringency, and for providing an antibacterial effect. See Panda, A. et al., Natural Product Radiance 5(4) 284-288 (2006).

In some preferred embodiments of the invention, the compositions include zinc oxide and/or zinc sulfate. In some such embodiments, the zinc oxide and/or zinc sulfate is in particulate form. It has been discovered in accordance with the present invention that while one would expect that zinc would have to be provided in a soluble state to be most effective, surprisingly, the incorporation of zinc oxide particles, which possess minimum solubility, in the composition of the invention provides the desired antibacterial and astringency properties, while, providing the additional benefit of avoiding the lingering unpleasant metallic and astringent taste of most zinc salts. In addition, while not wishing to be bound by a particular theory, because zinc sulfate is soluble whereas zinc oxide is not, it would be expected that zinc sulfate would be a more effective antimicrobial agent. Applicants have surprisingly found, however, that in the compositions of the invention, the two salts are possess roughly equivalent antimicrobial effectiveness, as demonstrated by the Examples below.

In some embodiments, the oral care compositions of the present disclosure also contain one or more cooling agents, for example camphor. A wide variety of cooling agents are amenable to the present invention. Typically, the cooling agent(s) is (are) present in an amount of from 0-2% by weight; for example from 0.1-1% by weight; for example about 0.5 by weight of the composition.

In some embodiments, the compositions of the invention contain one or more flavorants, useful for example to enhance taste of the composition. Any orally acceptable natural or synthetic flavorant can be used, including without limitation vanillin, sage, marjoram, parsley oil, spearmint oil, cinnamon oil, oil of wintergreen (methylsalicylate), peppermint oil, clove oil, bay oil, anise oil, eucalyptus oil, citrus oils, fruit oils and essences including those derived from lemon, orange, lime, grapefruit, apricot, banana, grape, apple, strawberry, cherry, pineapple, etc., bean- and nut-derived flavors such as coffee, cocoa, cola, peanut, almond, etc., adsorbed and encapsulated flavorants and the like. Also encompassed within flavorants herein are ingredients that provide fragrance and/or other sensory effect in the mouth, including cooling or warming effects. Such ingredients illustratively include menthol, menthyl acetate, menthyl lactate, camphor, eucalyptus oil, eucalyptol, anethole, eugenol, cassia, oxanone, .alpha.-irisone, propenyl guaiethol, thymol, linalool, benzaldehyde, cinnamaldehyde, N-ethyl-p-menthan-3-carboxamine, N,2,3-trimethyl-2-isopropylbutanamide, 3-(1-menthoxy)-propane-1,2-diol, cinnamaldehyde glycerol acetal (CGA), menthone glycerol acetal (MGA) and the like. The one or more flavorants are optionally present in a total amount of 0.01% to 5%, for example 0.1% to 2.5%, for example 0.1-1%; for example about 0.5%-0.6% by weight of the composition. Preferred flavorants include eugenol, for example in an amount of from 0.1% to 2%, for example 0.1% to 1%, for example 0.6% by weight of the composition; L-menthol, for example in an amount of from 0.1% to 4%, for example 0.1% to 2%, for example 1% by weight of the composition; and clove oil, for example in an amount of from 0.1% to 4%, for example 0.1% to 2%, for example 0.6% by weight of the composition. Other preferred flavorants include, without limitation, thymol, coriander oil, eucalyptus oil, dill oil, fennel oil, neem oil, lemon oil, and basil oil; each typically being present for example in an amount of from 0.1% to 4%, for example 0.1% to 2%, for example 1% to 2% by weight of the composition.

In preferred embodiments, the compositions of the disclosure contain at least one thickening agent, useful for example to impart a desired consistency and/or mouth feel to the composition. Any orally acceptable thickening agent can be used, including without limitation carbomers, also known as carboxyvinyl polymers, carrageenans, also known as Irish moss and more particularly 1-carrageenan (iota-carrageenan), cellulosic polymers such as hydroxyethylcellulose, carboxymethylcellulose (CMC) and salts thereof, e.g., CMC sodium, natural gums such as karaya, xanthan, gum arabic and tragacanth, colloidal magnesium aluminum silicate, colloidal silica and the like. One or more thickening agents are optionally present in a total amount of 0.01% to 15%, for example 0.1% to 10%, or 0.2% to 5%, or 3-4% by weight of the composition. In some preferred embodiments, the compositions include hydroxyethylcellulose and carboxymethylcellulose in a combined total amount of 3-4% by weight of the composition, and in a ratio of carboxymethylcellulose to hydroxyethylcellulose of from 1:1 to 1:2, for example from 1:1.4 to 1:1.8, for example about 1:1.6 by weight.

In some embodiments, the compositions of the invention comprise at least one surfactant, useful for example to compatibilize other components of the composition. Any orally acceptable nonionic surfactant is preferred,. Suitable nonionic surfactants include without limitation poloxamers, polyoxyethylene sorbitan esters, fatty alcohol ethoxylates, alkylphenol ethoxylates, tertiary amine oxides, tertiary phosphine oxides, dialkyl sulfoxides, polysorbates and the like. A suitable example is polysorbate 20 (Tween™ 20). The one or more surfactants are typically present in a total amount of 0.01% to 10%, for example 0.05% to 5% or 2% to 4%, for example 3%, by weight of the composition.

In some embodiments, the compositions of the invention comprise at least one solvent and/or humectant, useful for example to modulate the viscosity of the composition, provide body to the composition, and enhance sweetness. Any orally acceptable humectant can be used, including without limitation polyhydric alcohols such as glycerin, propylene glycol, sorbitol, xylitol or low molecular weight PEGs. Most humectants also function as sweeteners. The one or more humectants are preferably present in a total amount of 1% to 70%, for example 1% to 50%, 2% to 25%, or 10% to 20%, for example 15% by weight of the composition. In some preferred embodiments, the compositions comprise glycerin and propylene glycol in a combined total amount of 10%-20%; for example about 15%, by weight of the composition, and in a ratio of propylene glycol to glycerin of from 1:1 to 1:3, for example from 1:2, by weight.

In some embodiments, the compositions of the invention comprise at least one preservative. A wide variety of suitable preservatives are known, including, for example and not limitation, sodium benzoate, methyl paraben, propyl paraben, benzyl alcohol, ethanol, citric acid, potassium sorbate, salicylic acid, sorbic acid, inorganic sulfites, triclosan, and many flavors,

In some embodiments, the compositions of the invention comprise one or more additional ingredients such as sweeteners and colorants. Examples of suitable sweeteners include, for example and not limitation, sodium saccharin, stevia, sucralose, liquorice, and aspartame sodium. , colorants- zinc oxide, talc, titanium dioxide, pigment and dyes.

Colorants herein include pigments, dyes, lakes and agents imparting a particular luster or reflectivity such as pearling agents. A colorant can serve a number of functions, including for example to provide a white or light-colored coating on a dental surface, to act as an indicator of locations on a dental surface that have been effectively contacted by the composition, and/or to modify appearance, in particular color and/or opacity, of the composition to enhance attractiveness to the consumer. Any orally acceptable colorant can be used, including without limitation talc, mica, magnesium carbonate, calcium carbonate, magnesium silicate, magnesium aluminum silicate, silica, titanium dioxide, zinc oxide, red, yellow, brown and black iron oxides, ferric ammonium ferrocyanide, manganese violet, ultramarine, titaniated mica, bismuth oxychloride, pigments, dyes and the like. The one or more colorants are optionally present in a total amount of 0.001% to 20%, for example 0.01% to 10% or 0.1% to 5% by weight of the composition.

It will be recognized that various ingredients of the compositions of the invention can serve multiple purposes. For example, camphor functions as both a pain relief agent, and as a cooling agent. Clove oil, apart from its flavor properties, provides pain relief, anesthetic effect, antimicrobial properties and surprisingly acts as solubilizer for camphor. And propylene glycol can function both as a solvent (for example in helping to dissolve other components such as camphor), and as a humectant. Thus, the inclusion of a given ingredient in a list of a compounds having a particular function is not intended to limit the ingredient to that particular function.

### EXAMPLES

### Example 1 - Antibacterial activity of extracts against oral pathogens causing canker sores

A study was conducted to investigate the sensitivity of an oral bacterial and fungal strain toward two extracts: Licorice & Amla, with zinc oxide individually and in combination. Amla Extract and Liquorice Extract were obtained from Sabinsa Corporation, East Windsor, NJ. An Agar cup plate method was used wherein the test samples diffuse from the cup through an agar layer in a Petriplate to such an extent that the growth of added microorganisms is restricted entirely to a circular area or zone around the cavity containing the solution of an antibiotic substance. The antimicrobial activity is expressed as zone diameter in millimeters, which is measured by a scale.

Organisms: Staphylococcus aureus ATCC 6538 was employed as the bacterial strain in the study, and *Candida albicans* ATCC 10231 was employed as a fungal strain.

Preparation of extract suspensions: suspensions of all extracts were prepared by combining 100 mg of extract in 10 ml of DMSO diluent, for produce a test / stock solution with a concentration of 10 mg/ml (1%). Further dilutions were prepared from this stock solution to obtain concentrations of 1 mg/ml (0.1%) and 3 mg/ml (0.3%).

Preparation of test cultures: The inocula of the test strains were prepared from overnight cultures and the suspensions were adjusted to 0.5 McFarland standard turbidity (corresponding to 10⁸CFU/mL for bacteria & 10⁶CFU/mL for fungi).

Preparation of Agar plates (surface spread): Base layer was obtained by pouring around 20-30 ml of Trypticase soya Agar to obtain a thickness of 4 mm. It was then kept for solidification. A sterile swab was dipped into the inoculation tube. The swab was then rotated against the side of the tube (above the fluid level) using firm pressure, to remove excess fluid such that it was not dripping wet. TSA agar plates were then inoculated by streaking the swab three times over the entire agar surface & rotating the plate approximately 60 degrees each time to ensure an even distribution of the inoculum. Leaving the lid slightly ajar, the plate was then allowed to sit at room temperature at least 3 to 5 minutes, but no more than 15 minutes, for the surface of the agar plate to dry before proceeding to the next step.

### Experimental Procedure:

A Sterile cork borer was used to prepare 3 cups of 10mm diameter, in the medium of each Petri dish. The Petri dish was marked with the name of the extract & the three cups were marked with the 2 different concentrations, 1% and 3%, respectively. A suspension was prepared with equal concentrations of both the extracts to test the efficacy of these extracts in combination. 150 µl of the test suspension was then introduced with a micropipette in each of the wells. Sodium benzoate was used as positive control and DMSO was used as diluent control. All the plates were kept at room temperature for effective diffusion of the test sample and controls. The plates were later incubated at 37 ± 1°C for 24 hours and evaluated. The presence of definite zones around the cup of any size indicated antibacterial activity. The diameter of the zone of inhibition was measured and recorded. Zones above 8-10 mm were considered as significant.

The results are shown in Table 1 below:

**Table 1**

| Material Tested | In Candida | In S. aureus |
|---|---|---|
| Amla at 1% | 21 mm | 18 mm |
| Liquorice at 1% | 15 mm | 13 mm |
| Amla at 3% | 22 mm | 24 mm |
| Liquorice at 3% | 18 mm | 18 mm |

It can be seen that both Amla and Liquorice showed significant zones of inhibition when tested against S. Aureus and C Albicans. Since Amla and Liquorice are predominantly known for astringency and wound healing properties, respectively, it is both surprising and unexpected that both Amla and Liquorice showed significant inhibition for S. Aureus and C. Albicans. Accordingly, the compositions of the invention possess the added benefit of antibacterial and antifungal efficacies.

### Example 2 - Short Interval Kill Time (SIKT) In-vitro Test

The Short Interval Kill Time (SIKT) test was used to determine the in vitro short-term antimicrobial activity of compounds when tested in several formulations according to the invention. This test assesses the reduction of a microbial population of test organisms after exposure to the ulcer gel in-vitro. Test materials were mixed with bacterial inoculum for a selected time interval, after which the test system is neutralized and surviving bacteria enumerated. Dentifrice or other viscous or solid samples must be diluted. Bacterial reductions compared to water was used as the basis for expressing activity.

Staphylococcus aureus ATCC 6538 and C albicans ATCC 10231 were selected as the test organisms. Candida albicans is the most commonly implicated organism for oral thrush. Normal flora of the mouth and teeth includes Staphylococci. These two organisms are opportunistic pathogens that cause secondary infection. Three compositions (A-C) according to the invention were prepared as shown in Table 2 below. These compositions, and two commercial compositions, shown in Table 3, were subjected to the SIKT analysis.

**Table 2**

| Exemplary Compositions of the Invention | | | |
|---|---|---|---|
| Ingredients | Inventive Composition A (wt. %) | Inventive Composition B (wt. %) | Inventive Composition C (wt. %) |
| Hydroxyethylcellulose | 2.3 | 2.3 | 2.3 |
| Carboxymethylcellulose | 1.4 | 1.4 | 1.4 |
| Glycerin | 10.0 | 10.0 | 10.0 |
| Zinc Oxide | 1.0 | 1.0 | -- |
| Zinc Sulfate | -- | -- | 2.2 |
| Liquorice Extract | 1.0 | 2.5 | 1.0 |
| Amla Extract | 1.0 | 5.0 | 1.0 |
| Sodium Benzoate | 0.5 | 0.5 | 0.5 |
| Tween 20 | 3.0 | 3.0 | 3.0 |
| Propylene Glycol | 5.0 | 5.0 | 5.0 |
| L- Menthol | 1.0 | 1.0 | 1.0 |
| Camphor | 0.5 | 0.5 | 0.5 |
| Eugenol | 0.6 | 0.6 | 0.6 |
| Sodium Saccharin | 0.4 | 0.4 | 0.4 |
| Water | QS to 100 | QS to 100 | QS to 100 |

**Table 3**

| Commercial Mouth Ulcer Gel Formulations (ingredients as listed on product) | | | |
|---|---|---|---|
| **Commercial Formulation 1** | | **Commercial Formulation 2** | |
| Extracts | % w/w | Extracts | % w/w |
| Jati (jasminum grandiflorum) | 2.00 | Khadir (Acacia catechu) | 13.40 |
| Yashtimadhu (glycyrrhiza glabra) | 0.80 | Irimed (Acacia Farnesiana) | 13.40 |
| Triphala | 0.60 | Tagar (Valerina Wallichii) | 0.32 |
| Punarnava (Boerhaavia diffusa) | 0.60 | Rasna (Pluchea Lanceolata) | 0.16 |
| Oils | | Kushtha (Saussurea Lappa) | 0.48 |
| Lavanga (Syzygium aromaticum) | 0.50 | Lodhra (Symplocos Racemosa) | 0.30 |
| Vanatulsi (Ocimum basilicum) | 0.33 | Yashtimadhu (glycyrrhiza glabra) | 4.0 |
| Nimba (Azadirachta indica) | 0.17 | Karpoor (Cinnamomum Camphora) | 0.10 |
| Sodium Saccharin | 0.15 | Sharkara (Saccharum officinarum) 150 | 15.0 |

Inoculum Preparation: The inocula of the test strains were prepared from overnight cultures and the suspensions were adjusted to 0.5 McFarland standard turbidity (corresponding to 10⁸CFU/mL for bacteria & 10⁶ CFU/mL for fungi).

Sample Preparation: 9.0 g of each test sample was aseptically weighed into each of two sterile containers. 9.0 mL of sterile DI water was aseptically pipetted into a sterile container to serve as the test control.

Sample Inoculation (1:10): Each sample and control were inoculated with 1.0 mL of the prepared inoculum. Samples were stirred and mixed, and at the end of the selected contact time (i.e., 1 minute, 2 minutes or 5 minutes) 0.5 g was removed with a sterile stir rod and dispensed aseptically into 4.5 mL of Difco D/E Neutralizing Broth in a test tube. The D/E and product were well vortexed to insure neutralization of the antimicrobial.

Sample Testing: At each selected time interval, 1:10 dilutions (10-1 to 10-6) of each sample was prepared, using D/E broth (0.5 mL into 4.5 mL D/E). The samples were mixed well or vortexed. 1.0 mL of each dilution (10-1 to 10-6) was then pipetted into sterile Petri dishes. 15-20 mL of melted Modified Letheen Agar (MLA) was added, and the plates were swirled to disperse completely. After allowing the plates to solidify, they were inverted and incubated at 35°C for 48-72 hours, and the colonies counted.

Calculations: Two counts per sample point were averaged (A+B/2) to give mean CFU/g. Percent reduction was calculated by subtracting the averaged test sample CFU/g from the control CFU/g. This result was then divided by the control CFU x 100. Percent reduction was calculated by subtracting the averaged test sample CFU/g from the control CFU/g. This result was then divided by the control CFU x 100.

Results: In *S. aureus & C.albicans:* All three inventive compositions A-C listed in Table 2 had significant % kill as compared to Commercial Gel 2 starting from 1 min interval. On the other hand, their % kill was comparable to Commercial Gel 1. Details of results are attached in Tables 4 and 5, below:

**Table 4**

| SIKT Results for Exemplary Inventive Compositions vs Commercial Gels Using Test Organism S Aureus | | | | |
|---|---|---|---|---|
| Material Tested | | **1 minute** | **2 minutes** | **5 minutes** |
| | | Count (cfu/g) | | |
| Distilled Water (Control) | Control (cfu/g) | 223000000 | 178000000 | 98000000 |
| Composition A 1% extract + clove oil | Recovery (cfu/g) | 610000 | 370000 | 0 |
| | % kill | 99.73 | 99.79 | 100.00 |
| Composition B High extract + clove oil | Recovery (cfu/g) | 4900000 | 410000 | 1160 |
| | % kill | 97.80 | 99.77 | 100.00 |
| Composition C 1% extract + clove oil + Zinc sulfate | Recovery (cfu/g) | 470 | 100 | 0 |
| | % kill | 100.00 | 100.00 | 100.00 |
| Commercial Gel 2 | Recovery (cfu/g) | 60000000 | 32000000 | 9100000 |
| | % kill | 73.09 | 82.02247191 | 90.71 |
| Commercial Gel 1 | Recovery (cfu/g) | 1100000 | 840000 | 310000 |
| | % kill | 99.51 | 99.53 | 99.68 |

**Table 5**

| SIKT Results for Exemplary Inventive Compositions vs Commercial Gels Using Test Organism C Albicans | | | | |
|---|---|---|---|---|
| Material Tested | | **1 minute** | **2 minutes** | **5 minutes** |
| | | Count (cfu/g) | | |
| Distilled Water (Control) | Control (cfu/g) | 710000 | 640000 | 480000 |
| Composition A 1% extract + clove oil | Recovery (cfu/g) | 17000 | 0 | 0 |
| | % kill | 97.61 | 100.00 | 100.00 |
| Composition B High extract + clove oil | Recovery (cfu/g) | 0 | 0 | 0 |
| | % kill | 100.00 | 100.00 | 100.00 |
| Composition C 1% extract + clove oil + Zinc sulfate | Recovery (cfu/g) | 560 | 200 | 0 |
| | % kill | 99.92 | 99.97 | 100.00 |
| Commercial Gel 2 | Recovery (cfu/g) | 77000 | 35000 | 1800 |
| | % kill | 89.15 | 94.53 | 99.63 |
| Commercial Gel 1 | Recovery (cfu/g) | 3000 | 0 | 0 |
| | % kill | 99.58 | 100.00 | 100.00 |

Results: It can be seen that the antibacterial properties of the compositions of the invention will help to heal mouth ulcers quickly by preventing the oral pathogenic bacteria from proliferating and causing mucosal damage. It will also prevent incidence of secondary infection.

### Example 3 - Short Interval Kill Time (SIKT) In-vitro Test For Further Exemplary Compositions of the Invention

The SIKT test as described above was also performed for compositions of the invention D,E and F, having the compositions shown in Table 6 below:

**Table 6**

| Ingredients | Inventive Composition D (wt. %) | Inventive Composition E (wt. %) | Inventive Composition F (wt. %) |
|---|---|---|---|
| Natrosol M250 | 2.3 | 2.0 | 2.3 |
| PEG 600 | 3.0 | 3.0 | 3.0 |
| Glycerin | 10.0 | 10.0 | 10.0 |
| Zinc Oxide | --- | 1.0 | 0.5 |
| Zinc Sulfate | 2.2 | -- | 1.1 |
| Liquorice Extract | 1.0 | 1.0 | 1.0 |
| Amla Extract | 1.0 | 1.0 | 1.0 |
| Sodium Benzoate | 0.5 | 0.5 | 0.5 |
| Croduret 40 | 2.0 | 3.0 | 2.0 |
| Propylene Glycol | 5.0 | 5.0 | 5.0 |
| L- Menthol | 1.0 | 1.0 | 1.0 |
| Camphor | 0.5 | 0.5 | 0.5 |
| Clove oil | 0.6 | 0.6 | 0.6 |
| Sodium Saccharin | 0.5 | 0.5 | 0.5 |
| Coconut Oil Refined | --- | 3.0 | --- |
| Purified Water | 70.4 | 67.9 | 71 |

Tables 7 and 8 below show the results for test organisms S. aureus and C. albicans, respectively:

**Table 7**

| SIKT Results for Exemplary Inventive Compositions vs Commercial Gels Using Test Organism S Aureus | | | | |
|---|---|---|---|---|
| Material Tested | | **1 minute** | **2 minutes** | **5 minutes** |
| | | Count (cfu/g) | | |
| Distilled Water (Control) | Control (cfu/g) | 75000000 | 260000000 | 13000000 |
| Composition D 1% extract + Natrosol + Zinc Sulfate | Recovery (cfu/g) | 40000 | 1900 | 0 |
| | % kill | 99.95 | 99.99 | 100.00 |
| Composition E 1% extract + Natrosol + ZnO + Coconut Oil | Recovery (cfu/g) | 160000 | 15000 | 1300 |
| | % kill | 97.79 | 99.94 | 99.99 |
| Composition F Zinc Sulfate + Zinc Oxide | Recovery (cfu/g) | 210000 | 21000 | 3900 |
| | % kill | 77.3 | 81.92 | 93.08 |
| Commercial Gel 2 | Recovery (cfu/g) | 17000000 | 4700000 | 900000 |
| | % kill | 77.33 | 81.92 | 93.08 |
| Commercial Gel 1 | Recovery (cfu/g) | 660000 | 57000 | 310000 |
| | % kill | 99.12 | 99.78 | 97.62 |

**Table 8**

| SIKT Results for Exemplary Inventive Compositions vs Commercial Gels Using Test Organism C Albicans | | | | |
|---|---|---|---|---|
| Material Tested | | **1 minute** | **2 minutes** | **5 minutes** |
| | | Count (cfu/g) | | |
| Distilled Water (Control) | Control (cfu/g) | 430000 | 420000 | 370000 |
| Composition D 1% extract + Natrosol + Zinc Sulfate | Recovery (cfu/g) | 0 | 0 | 0 |
| | % kill | 100.00 | 100.00 | 100.00 |
| Composition E 1% extract + Natrosol + ZnO + Coconut Oil | Recovery (cfu/g) | 0 | 0 | 0 |
| | % kill | 100.00 | 100.00 | 100.00 |
| Composition F Zinc Sulfate + Zinc Oxide | Recovery (cfu/g) | 0 | 0 | 0 |
| | % kill | 100.00 | 100.00 | 100.00 |
| Commercial Gel 2 | Recovery (cfu/g) | 510000 | 22000 | 7000 |
| | % kill | 88.14 | 94.76 | 98.11 |
| Commercial Gel 1 | Recovery (cfu/g) | 2800 | 0 | 0 |
| | % kill | 99.35 | 100.00 | 100.00 |

Results: All formulations D-F are superior to Commercial Gel 2, and comparable to Commercial Gel 1. The combination of zinc sulfate and zinc oxide did not show a superior kill as compared to only zinc oxide. Additionally, it is surprising and unexpected that all three formulations D-F have roughly equivalent kill properties, irrespective of the solubility of zinc salt.

## Claims

1. An oral care composition comprising: Amla extract; Liquorice Extract; a zinc ion source, and optionally, camphor, in a pharmaceutically acceptable carrier.

2. The oral care composition of claim 1, wherein the composition is an oral gel.

3. The oral care composition of claim 1 or claim 2, wherein:
the Amla extract is present in the composition in an amount of from 0.5% to 8% by weight, for example from 1% to 5% by weight;
the Liquorice extract is present in the composition in an amount of from 0.1% to 5% by weight, for example from 1% to 3% by weight;
the zinc source is present in the composition in an amount sufficient to provide zinc ions in an amount of from 0.1% to 2% by weight for example from 0.5% to 1.2% by weight; and
the camphor, is present in the composition in an amount of from 0% to 2% by weight.

4. The oral care composition of any preceding claim, wherein the zinc ion source is zinc acetate, zinc citrate, zinc gluconate, zinc glycinate, zinc oxide, zinc sulfate and sodium zinc citrate, or a combination of two or more thereof; for example zinc oxide, or zinc sulfate, or a combination thereof; wherein the zinc oxide is optionally in particulate form.

5. The oral care composition of any preceding claim, further comprising one or more flavorants; for example eugenol, L-menthol, clove oil, thymol, coriander oil, eucalyptus oil, fennel oil, neem oil, lemon oil, coconut oil, and/or basil oil;
for example wherein clove oil is present in an amount of from 0.1% to 2%; and L-menthol is present in an amount of from 0.1% to 4%, by weight of the composition; or for example wherein clove oil is present in an amount of from 0.1% to 1%; and L-menthol is present in an amount of from 0.1% to 2%, by weight of the composition.

6. The oral care composition of any preceding claim, wherein eugenol is present in an amount of from 0.1% to 2%; and L-menthol is present in an amount of from 0.1% to 4%, by weight of the composition; for example wherein eugenol is present in an amount of from 0.1% to 1%; and L-menthol is present in an amount of from 0.1% to 2%, by weight of the composition.

7. The oral care composition of any preceding claim, wherein coconut oil is present in an amount of from 0.1% to 5% by weight of the composition; for example from 1% to 4%, by weight of the composition.

8. The oral care composition of any preceding claim, wherein the pharmaceutically acceptable carrier comprises one or more thickeners, for example selected from modified celluloses, hydroxyethylcellulose, carboxymethylcellulose, PMC, Veegum, Xanthan, hydropropylcellulose, methylcellulose, carrageenan, tragacanth and combinations of two or more thereof.

9. The oral care composition of any preceding claim, further comprising a nonionic surfactant, for example selected from Croduret 40, a PEG, PEG 600, polysorbate 20 (TweenTM 20), polysorbate 80 (TweenTM 80), PEG 6000, and Poloxamers, and combinations of two or more thereof, for example wherein the nonionic surfactant is polysorbate 20 (TweenTM 20), alone or in combination with another nonionic surfactant.

10. The oral care composition of any preceding claim, further comprising one or more solvents, for example wherein the solvents comprise propylene glycol.

11. The oral care composition of any preceding claim, further comprising one or more humectants and/or preservatives; for example wherein the humectant comprises glycerin; and for example wherein the preservatives are selected from sodium benzoate, methyl paraben, propyl paraben, benzyl alcohol, ethanol, citric acid, potassium sorbate, salicylic acid, sorbic acid, inorganic sulfites, triclosan, flavors and combinations of two or more thereof.

12. The oral care composition of any preceding claim, further comprising one or more additional ingredients selected from sweeteners and colorants; for example wherein the sweeteners are selected from stevia, sucralose, liquorice, aspartame sodium and combinations of two or more thereof; and the colorants are selected from zinc oxide, talc, titanium dioxide, pigments, dyes and combinations of two or more thereof.

13. A composition of any preceding claim 1-12 for use in treating ulcerous conditions of the oral cavity.

14. The composition for use of claim 13 wherein the ulcerous condition is mouth ulcers or canker sores.

15. A method for preparing a composition of any preceding claim 1-14, comprising combining together Amla extract, Liquorice Extract, and a zinc ion source, in a pharmaceutically acceptable carrier.

## Patentansprüche

1. Mundpflegezusammensetzung umfassend: Amlaextrakt, Lakritzextrakt, eine Zinkionenquelle und gegebenenfalls Kampfer in einem pharmazeutisch annehmbaren Träger.

2. Mundpflegezusammensetzung nach Anspruch 1, worin die Zusammensetzung ein orales Gel ist.

3. Mundpflegezusammensetzung nach Anspruch 1 oder Anspruch 2, worin:
das Amlaextrakt liegt in der Zusammensetzung in einer Menge von 0,5 bis 8 Gew.-% vor, beispielsweise von 1 bis 5 Gew.-%;
der Lakritzextrakt liegt in der Zusammensetzung in einer Menge von 0,1 bis 5 Gew.-% vor, beispielsweise von 1 bis 3 Gew.-%;
die Zinkquelle liegt in der Zusammensetzung in einer Menge vor, die ausreicht, um Zinkionen in einer Menge von 0,1 bis 2 Gew.-% bereitzustellen, beispielsweise von 0,5 bis 1,2 Gew.-%; und
das Kampfer liegt in der Zusammensetzung in einer Menge von 0 bis 2 Gew.-% vor.

4. Mundpflegezusammensetzung nach irgendeinem vorhergehenden Anspruch, worin die Zinkionenquellen Zinkacetat, Zinkcitrat, Zinkgluconat, Zinkglycinat, Zinkoxid, Zinksulfat und Natriumzinkcitrat oder eine Kombination von zwei oder mehreren davon ist; beispielsweise Zinkoxid oder Zinksulfat oder eine Kombination davon, wobei das Zinkoxid gegebenenfalls in Partikelform vorliegt.

5. Mundpflegezusammensetzung nach irgendeinem vorhergehenden Anspruch, weiterhin umfassend ein oder mehrere Duftstoffe, beispielsweise Eugenol, L-Menthol, Nelkenöl, Thymol, Korianderöl, Eukalyptusöl, Fenchelöl, Neemöl, Zitronenöl, Kokosnussöl und/oder Basilikumöl;
beispielsweise liegt das Nelkenöl in einer Menge von 0,1 bis 2% vor und L-Menthol liegt in einer Menge von 0,1 bis 4 Gew.-% der Zusammensetzung vor oder beispielsweise liegt das Nelkenöl in einer Menge von 0,1 bis 1% vor und L-Menthol liegt in einer Menge von 0,1 bis 2 Gew.-% der Zusammensetzung vor.

6. Mundpflegezusammensetzung nach irgendeinem vorhergehenden Anspruch, worin Eugenol in einer Menge von 0,1 bis 2% vorliegt; und L-Menthol liegt in einer Menge von 0,1 bis 4 Gew.-% der Zusammensetzung vor, beispielsweise liegt das Eugenol in einer Menge von 0,1% bis 1%vor und L-Menthol liegt in einer Menge von 0,1 bis 2 Gew.-% der Zusammensetzung vor.

7. Mundpflegezusammensetzung nach irgendeinem vorhergehenden Anspruch, worin das Kokosnussöl in einer Menge von 0,1 bis 5 Gew.-% der Zusammensetzung vorliegt, beispielsweise 1 bis 4 Gew.-% der Zusammensetzung.

8. Mundpflegezusammensetzung nach irgendeinem vorhergehenden Anspruch, worin der pharmazeutisch annehmbare Träger ein oder mehrere Verdickungsmittel umfasst, beispielsweise ausgewählt aus modifizierten Cellulosen, Hydroxyethylcellulose, Carboxymethylcellulose, PMC, Veegum, Xanthan, Hydroxypropylcellulose, Methylcellulose, Carrageenan, Tragacauth und Kombinationen aus zwei oder mehreren davon.

9. Mundpflegezusammensetzung nach irgendeinem vorhergehenden Anspruch, weiterhin umfassend ein nichtionisches Tensid, beispielsweise ausgewählt aus Croduret 40, einem PEG, PEG 600, Polysorbat 20 (Tween TM 20), Polysorbat 80 (Tween TM 80), PEG 6000 und Poloxameren und Kombinationen von zwei oder mehreren davon, beispielsweise wobei das nichtionische Tensid Polysorbat 20 (Tween TM 20) ist, alleine oder in Kombination mit einem anderen nichtionischen Tensid.

10. Mundpflegezusammensetzung nach irgendeinem vorhergehenden Anspruch, weiterhin umfassend ein oder mehrere Lösemittel, beispielsweise worin die Lösemittel Propylenglycol umfassen.

11. Mundpflegezusammensetzung nach irgendeinem vorhergehenden Anspruch, weiterhin umfassend ein oder mehrere Befeuchtungsmittel und/oder Konservierungsmittel; beispielsweise worin das Befeuchtungsmittel Glycerin umfasst und beispielsweise worin die Konservierungsmittel ausgewählt sind aus Natriumbenzoat, Methylparaben, Propylparaben, Benzylalkohol, Ethanol, Zitronensäure, Kaliumsorbat, Salicylsäure, Sorbinsäure, anorganischen Sulfiten, Triclosan, Geschmacksstoffe und Kombinationen aus zwei oder mehreren davon.

12. Mundpflegezusammensetzung nach irgendeinem vorhergehenden Anspruch, weiterhin umfassend ein oder mehrere zusätzliche Bestandteile, ausgewählt aus Süßungsmitteln und Färbungsmitteln, beispielsweise worin die Süßstoffsmittel ausgewählt sind aus Stevia, Sucralose, Lakritz, Aspartamnatrium und Kombinationen aus zwei oder mehreren davon und die Farbstoffe sind ausgewählt aus Zinkoxid, Talkum, Titandioxid, Pigmenten, Farbstoffen und Kombinationen aus zwei oder mehreren davon.

13. Zusammensetzung nach irgendeinem vorhergehenden Anspruch zur Verwendung bei der Behandlung von geschwürartigen Zuständen der Mundhöhle.

14. Zusammensetzung zur Verwendung nach Anspruch 13 worin die geschwürartigen Zustände Mundgeschwüre oder Krebsgeschwüre sind.

15. Verfahren zur Herstellung einer Zusammensetzung nach irgendeinem vorhergehenden Ansprüche 1 bis 14 umfassend das Zusammenkombinieren von Amlaextrakt, Lakritzextrakt und einer Zinkionenquelle in einem pharmazeutisch annehmbaren Träger.

## Revendications

1. Composition de soin buccal comprenant : un extrait d'Amla ; un extrait de réglisse ; une source d'ions zinc et, éventuellement, du camphre, dans un support pharmaceutiquement acceptable.

2. Composition de soin buccal selon la revendication 1, dans laquelle la composition est un gel buccal.

3. Composition de soin buccal selon la revendication 1 ou la revendication 2, dans laquelle :
l'extrait d'Amla est présent dans la composition en une quantité de 0,5 % à 8 % en poids, par exemple de 1 % à 5 % en poids ;
l'extrait de réglisse est présent dans la composition en une quantité d'environ 0,1 % à 5 % en poids, par exemple de 1 % à 3 % en poids ;
la source de zinc est présente dans la composition en une quantité suffisante pour fournir des ions zinc en une quantité de 0,1 % à 2 % en poids, par exemple de 0,5 % à 1,2 % en poids ; et
le camphre, est présent dans la composition en une quantité de 0 % à 2 % en poids.

4. Composition de soin buccal selon l'une quelconque des revendications précédentes, dans laquelle la source d'ions zinc est l'acétate de zinc, le citrate de zinc, le gluconate de zinc, le glycinate de zinc, l'oxyde de zinc, le sulfate de zinc et le citrate sodique de zinc, ou une combinaison de deux ou plus de ceux-ci ; par exemple l'oxyde de zinc, ou le sulfate de zinc, ou une combinaison de ceux-ci ; dans laquelle l'oxyde de zinc est éventuellement sous forme particulaire.

5. Composition de soin buccal selon l'une quelconque des revendications précédentes, comprenant en outre un ou plusieurs aromatisants ; par exemple l'eugénol, le L-menthol, l'essence de clou de girofle, le thymol, l'essence de coriandre, l'essence d'eucalyptus, l'essence de fenouil, l'essence de neem, l'essence de citron, l'essence de noix de coco et/ou l'essence de basilic ; par exemple dans laquelle l'essence de clou de girofle est présente en une quantité de 0,1 % à 2 % ; et le L-menthol est présent en une quantité de 0,1 % à 4 %, en poids de la composition ; ou, par exemple, dans laquelle l'essence de clou de girofle est présente en une quantité de 0,1 % à 1 % ; et le L-menthol est présent en une quantité de 0,1 % à 2 %, en poids de la composition.

6. Composition de soin buccal selon l'une quelconque des revendications précédentes, dans laquelle l'eugénol est présent en une quantité de 0,1 % à 2 % ; et le L-menthol est présent en une quantité de 0,1 % à 4 %, en poids de la composition ; par exemple, dans laquelle l'eugénol est présent en une quantité de 0,1 % à 1 % ; et le L-menthol est présent en une quantité de 0,1 % à 2 %, en poids de la composition.

7. Composition de soin buccal selon l'une quelconque des revendications précédentes, dans laquelle l'essence de noix de coco est présente en une quantité de 0,1 % à 5 % en poids de la composition ; par exemple de 1 % à 4 %, en poids de la composition.

8. Composition de soin buccal selon l'une quelconque des revendications précédentes, dans laquelle le support pharmaceutiquement acceptable comprend un ou plusieurs épaississants, choisis par exemple parmi les celluloses modifiées, l'hydroxyéthylcellulose, la carboxyméthylcellulose, le PMC, le Veegum, le Xanthane, l'hydropropylcellulose, la méthylcellulose, le carraghénane, l'adragante et des combinaisons de deux ou plus de ceux-ci.

9. Composition de soin buccal selon l'une quelconque des revendications précédentes, comprenant en outre un tensioactif non ionique, choisi par exemple parmi le Croduret 40, un PEG, le PEG 600, le polysorbate 20 (Tween TM 20), le polysorbate 80 (Tween TM 80), le PEG 6000 et les Poloxamères, et des combinaisons de deux ou plus de ceux-ci, par exemple dans laquelle le tensioactif non ionique est le polysorbate 20 (Tween TM 20), seul ou en combinaison avec un autre tensioactif non ionique.

10. Composition de soin buccal selon l'une quelconque des revendications précédentes, comprenant en outre un ou plusieurs solvants, par exemple dans laquelle les solvants comprennent le propylène glycol.

11. Composition de soin buccal selon l'une quelconque des revendications précédentes, comprenant en outre un ou plusieurs agents humectants et/ou conservateurs ; par exemple, dans laquelle l'agent humectant comprend la glycérine ; et par exemple, dans laquelle les conservateurs sont choisis parmi le benzoate de sodium, le méthylparaben, le propylparaben, l'alcool benzylique, l'éthanol, l'acide citrique, le sorbate de potassium, l'acide salicylique, l'acide sorbique, les sulfites inorganiques, le triclosan, les arômes et des combinaisons de deux ou plus de ceux-ci.

12. Composition de soin buccal selon l'une quelconque des revendications précédentes, comprenant en outre un ou plusieurs ingrédients supplémentaires choisis parmi les édulcorants et les colorants ; par exemple, dans laquelle les édulcorants sont choisis parmi la stévia, le sucralose, la réglisse, l'aspartame sodique et des combinaisons de deux ou plus de ceux-ci ; et les colorants sont choisis parmi l'oxyde de zinc, le talc, le dioxyde de titane, des pigments, des colorants et des combinaisons de deux ou plus de ceux-ci.

13. Composition selon l'une quelconque des revendications 1 à 12 précédentes, pour une utilisation dans le traitement d'affections ulcéreuses de la cavité buccale.

14. Composition pour une utilisation selon la revendication 13, dans laquelle l'affection ulcéreuse est un ulcère de la bouche ou des aphtes.

15. Procédé de préparation d'une composition selon l'une quelconque des revendications 1 à 14 précédentes, comprenant la combinaison ensemble d'un extrait d'Amla, d'un extrait de réglisse et d'une source d'ions zinc, dans un support pharmaceutiquement acceptable.
